# EUROPEAN PATENT APPLICATION

(11) **EP 4 609 781 A1**
(43) Date of publication of application: **03.09.2025**
(21) Application number: 22963175.9
(22) Date of filing: 28.10.2022
(51) Int. Cl.: A61B 5/00

(54) **WEARABLE MOBILE MONITORING DEVICE, MONITORING SYSTEM, AND DATA TRANSMISSION METHOD**

(71) Applicant: Shenzhen Mindray Bio-Medical Electronics Co., Ltd., Shenzhen, Guangdong 518057 (CN)
(72) Inventor: CEN, Jian, Shenzhen, Guangdong 518057 (CN); QING, Lei, Shenzhen, Guangdong 518057 (CN); LI, Liya, Shenzhen, Guangdong 518057 (CN); XU, Li, Shenzhen, Guangdong 518057 (CN); LIU, Zhonghua, Shenzhen, Guangdong 518057 (CN); LUO, Hanyuan, Shenzhen, Guangdong 518057 (CN)
(74) Representative: KIPA AB
(86) International application number: PCT/CN2022/128350
(87) International publication number: WO 2024/087193

(57) **Abstract**

A wearable mobile monitoring device (100), a monitoring system (20), and a data transmission method (600). The device (100) comprises a sensor (110), a processor (120) and a wireless communication unit (130). The sensor (110) is used to acquire a signal representing at least one physiological sign parameter of a human body; the processor (120) is used to obtain monitoring data on the basis of the signal acquired by the sensor (110); and the wireless communication unit (130) is controlled to establish a communication connection with a monitoring device (200), and transmit monitoring data to the monitoring device (200). The data transmission method comprises: under a first preset condition, transmitting monitoring data corresponding to a signal acquired at a first acquisition frequency in a first transmission mode (S631); under a second preset condition, transmitting the monitoring data in a second transmission mode (S632); when the monitoring data is transmitted in the second transmission mode, if a third preset condition is detected, switching to a third transmission mode to transmit monitoring data corresponding to a signal acquired at the third acquisition frequency, and switching back to the second transmission mode when a transmission duration after switching to the third transmission mode reaches a first preset duration threshold, and the first preset condition is not detected (S633).

## Description

### TECHNICAL FIELD

The disclosure relates to a technical field of a medical monitoring device, and in particular to a wearable mobile monitoring device, a monitoring system, and a data transmission method.

### BACKGROUND

For a patient in a postoperative recovery period, it is clinically recommended that for the patient to get out of bed and move around as soon as possible to promote recovery. However, there is a risk that the medical condition of the patient may change during the postoperative recovery period, and this risk increases during a movement. Therefore, various wireless monitoring devices have emerged to monitor changes in vital signs of patient during a movement. In order to balance device battery life and continuous real-time monitoring for ensuring that, the risk that the medical condition of the patient changes, is identified in a timely manner, current wireless monitoring devices are often large in size and provide poor wearing comfort for patient.

### SUMMARY

This disclosure provides a wearable mobile monitoring device, a monitoring system and a data transmission method, which are capable of solving an above problem.

According to an aspect of this disclosure, a wearable mobile monitoring device is provided, including a sensor, a processor and a wireless communication unit; wherein the sensor is configured to acquire a signal which represents at least one vital sign parameter of a human body; the processor is configured to perform following operations: obtaining monitoring data which corresponds to the signal based on the signal which is acquired by the sensor; controlling the wireless communication unit to transmit the monitoring data to a monitoring device which is separate from the human body, through a communicative connection which is established between the wireless communication unit and the monitoring device;
wherein transmitting the monitoring data to the monitoring device includes:
under a first preset condition, transmitting, in a first transmission mode, the monitoring data which corresponds to the signal which is acquired at a first acquisition frequency;
under a second preset condition, transmitting, in a second transmission mode, the monitoring data which corresponds to the signal which is acquired at a second acquisition frequency; wherein a frequency for transmitting the monitoring data in the first transmission mode is higher than a frequency for transmitting the monitoring data in the second transmission mode, and/or the first acquisition frequency is higher than the second acquisition frequency;
when the monitoring data is transmitted in the second transmission mode; if a third preset condition is detected, switching to a third transmission mode in which mode the monitoring data, which corresponds to the signal which is acquired at a third acquisition frequency, is transmitted, and further switching back to the second transmission mode, when a transmission duration after switching to the third transmission mode reaches a first preset time threshold and the first preset condition is not detected; wherein a frequency for transmitting the monitoring data in the third transmission mode is higher than the frequency for transmitting the monitoring data in the second transmission mode, and/or the third acquisition frequency is higher than the second acquisition frequency.

According to another aspect of this disclosure, a monitoring system is provided, which includes a wearable mobile monitoring device which is worn on a human body, and a monitoring device which is separate from the human body;
wherein the mobile monitoring device is configured to detect monitoring data of the human body; the monitoring device is configured to acquire and display the monitoring data; the mobile monitoring device is further configured to perform following operations: transmitting the monitoring data to the monitoring device through a communicative connection which is established between the mobile monitoring device and the monitoring device; wherein transmitting the monitoring data to the monitoring device, includes:
under a first preset condition, transmitting, in a first transmission mode, the monitoring data which is acquired at a first acquisition frequency;
under a second preset condition, transmitting, in a second transmission mode, the monitoring data which is acquired at a second acquisition frequency; wherein a frequency for transmitting the monitoring data in the first transmission mode is higher than a frequency for transmitting the monitoring data in the second transmission mode, and/or the first acquisition frequency is higher than the second acquisition frequency;
when the monitoring data is transmitted in the second transmission mode; if a third preset condition is detected, switching to a third transmission mode in which mode the monitoring data, which is acquired at a third acquisition frequency, is transmitted, and further switching back to the second transmission mode, when a transmission duration after switching to the third transmission mode reaches a first preset time threshold and the first preset condition is not detected; wherein a frequency for transmitting the monitoring data in the third transmission mode is higher than the frequency for transmitting the monitoring data in the second transmission mode, and/or the third acquisition frequency is higher than the second acquisition frequency.

According to another aspect of this disclosure, a data transmission method is provided, which is applied to a wearable mobile monitoring device and includes:
acquiring a signal which represents at least one physiological sign parameter of a human body;
obtaining monitoring data based on the signal; and
transmitting the monitoring data to a monitoring device which is separate from the human body, through a communicative connection which is established between the mobile monitoring device and the monitoring device;
wherein transmitting the monitoring data to the monitoring device, includes:
   under a first preset condition, transmitting, in a first transmission mode, the monitoring data which is acquired at a first acquisition frequency;
   under a second preset condition, transmitting, in a second transmission mode, the monitoring data which is acquired at a second acquisition frequency; wherein a frequency for transmitting the monitoring data in the first transmission mode is higher than a frequency for transmitting the monitoring data in the second transmission mode, and/or the first acquisition frequency is higher than the second acquisition frequency;
   when the monitoring data is transmitted in the second transmission mode; if a third preset condition is detected, switching to a third transmission mode in which mode the monitoring data, which is acquired at a third acquisition frequency, is transmitted, and further switching back to the second transmission mode, when a transmission duration after switching to the third transmission mode reaches a first preset time threshold and the first preset condition is not detected; wherein a frequency for transmitting the monitoring data in the third transmission mode is higher than the frequency for transmitting the monitoring data in the second transmission mode, and/or the third acquisition frequency is higher than the second acquisition frequency.

In the wearable mobile monitoring device, monitoring system and data transmission method of this disclosure, after obtaining the monitoring data of the human body, the mobile monitoring device is capable of transmitting the monitoring data to the monitoring device which is separate from the human body through different transmission modes. These different transmission modes are capable of satisfying a scenario with a high monitoring requirement, a scenario with a low power consumption requirement, and a scenario that requires temporary mode switching, so as to satisfy various complex scenarios while minimizing power consumption.

### BRIEF DESCRIPTION OF THE DRAWINGS

FIG. 1 is a structural block diagram of a wearable mobile monitoring device according to an embodiment of this disclosure.
FIG. 2 is a structural block diagram of a monitoring system according to an embodiment of this disclosure.
FIG. 3 is a schematic diagram showing that a wearable mobile monitoring device transmits monitoring data to a monitoring device which is separate from a human body in different transmission modes, in a monitoring system according to an embodiment of this disclosure.
FIG. 4 is a schematic diagram showing an example in which countdown indication information is displayed on a monitoring device in a monitoring system according to an embodiment of this disclosure.
FIG. 5 is a schematic diagram showing another example in which countdown indication information is displayed on a monitoring device in a monitoring system according to an embodiment of this disclosure.
FIG. 6 is a schematic flowchart for a data transmission method according to an embodiment of this disclosure.

### DESCRIPTION OF THE PREFERRED EMBODIMENTS

In order to make the objectives, technical solutions and advantages of this disclosure more obvious, exemplary embodiments according to this disclosure will be described in detail below with reference to the accompanying drawings. Obviously, the described embodiments are only some embodiments of this disclosure, rather than all embodiments of this disclosure. It should be understood that this disclosure is not limited to the example embodiments described herein. Based on the embodiments of this disclosure described in this disclosure, all other embodiments obtained by those skilled in the art without creative work should fall within the protection scope of this disclosure.

In the following description, a large number of specific details are provided to provide a more thorough understanding of this disclosure. However, it is evident to those skilled in the art that this disclosure can be implemented without the need for one or more of these details. In other examples, in order to avoid confusion with this disclosure, some well-known technical characteristics in this field are not described.

It should be understood that this disclosure can be implemented in different forms and should not be limited to the embodiments proposed here. On the contrary, providing these embodiments make the disclosure thorough and complete, and fully convey the scope of this disclosure to those skilled in the art.

The purpose of the terminology used here is only to describe specific embodiments and is not a limitation of this disclosure. When used herein, the singular forms of "an", "a", and "the/said" are also intended to include the plural form, unless the context clearly indicates another way. It should also be understood that the terms, such as "including", "and/or", "consisting of", when used in this disclosure, determine a presence of a characteristic, integer, step, operation, component, and/or element, but do not exclude a presence or addition of one or more other characteristics, integers, steps, operations, components, and/or elements. When used here, the term "and/or" includes any and all combinations of related listed items.

In order to thoroughly understand this disclosure, detailed steps and detailed structures will be presented in the following description to illustrate the technical solution proposed in this disclosure. The preferred embodiments of this disclosure are described in detail below. However, in addition to these detailed descriptions, this disclosure may also have other implementation methods.

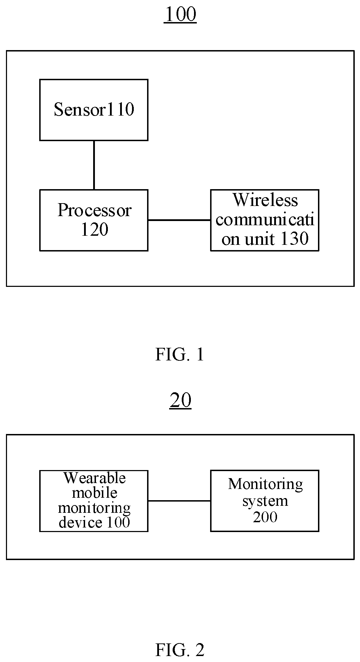

FIG. 1 is a structural block diagram of a wearable mobile monitoring device according to an embodiment of this disclosure. As shown in FIG. **1****,** the wearable mobile monitoring device 100 includes a sensor **110,** a processor 120, and a wireless communication unit 130. The sensor 110 is configured to acquire a signal which represents at least one vital sign parameter of a human body; the processor 120 is configured to perform following operations: obtaining monitoring data which corresponds to the signal based on the signal which is acquired by the sensor 110; controlling the wireless communication unit 130 to transmit the monitoring data to a monitoring device which is separate from the human body, through a communicative connection which is established between the wireless communication unit and the monitoring device. Wherein, transmitting the monitoring data to the monitoring device includes: under a first preset condition, transmitting, in a first transmission mode, the monitoring data which corresponds to the signal which is acquired at a first acquisition frequency; under a second preset condition, transmitting, in a second transmission mode, the monitoring data which corresponds to the signal which is acquired at a second acquisition frequency; wherein a frequency for transmitting the monitoring data in the first transmission mode is higher than a frequency for transmitting the monitoring data in the second transmission mode, and/or the first acquisition frequency is higher than the second acquisition frequency; when the monitoring data is transmitted in the second transmission mode; if a third preset condition is detected, switching to a third transmission mode in which mode the monitoring data, which corresponds to the signal which is acquired at a third acquisition frequency, is transmitted, and further switching back to the second transmission mode, when a transmission duration after switching to the third transmission mode reaches a first preset time threshold and the first preset condition is not detected; wherein, a frequency for transmitting the monitoring data in the third transmission mode is higher than the frequency for transmitting the monitoring data in the second transmission mode, and/or the third acquisition frequency is higher than the second acquisition frequency.

In an embodiment of this disclosure, after acquiring the monitoring data, the wearable mobile monitoring device 100 transmits the monitoring data to a monitoring device which is separate from the human body through a wireless communicative connection. In different situations, the monitoring data may be acquired at different acquisition frequencies, and may also be transmitted in different transmission modes. For example, under a first preset condition, the monitoring data which is acquired at a first acquisition frequency is transmitted in a first transmission mode; under a second preset condition, the monitoring data which is acquired at a second acquisition frequency is transmitted in a second transmission mode; wherein a frequency for transmitting the monitoring data in the first transmission mode is higher than a frequency for transmitting the monitoring data in the second transmission mode, and/or the first acquisition frequency is higher than the second acquisition frequency. Therefore, according to the embodiment of this disclosure, the mobile monitoring device 100 is capable of acquiring monitoring data at different acquisition frequencies, and also capable of adopting different transmission modes to transmit the monitoring data to the monitoring device, so as to satisfy transmission requirements under different conditions. Specifically, compared with the second acquisition frequency, the first acquisition frequency is higher, therefore is capable of satisfying a scenario with a higher requirement for human body monitoring, a scenario which does not pay much attention to power consumption, and the likes. Compared with the first acquisition frequency, the second acquisition frequency is lower, therefore is capable of satisfying a scenario with a lower requirement for human body monitoring, a scenario which pays more attention to power consumption, and the likes. Similarly, compared with the second transmission mode, the first transmission mode transmits the monitoring data at a higher frequency, therefore is capable of satisfying a scenario with a higher requirement for human body monitoring, a scenario where a communication environment is suitable for low-power data transmission, a scenario which does not pay much attention to power consumption, and the likes. Compared with the first transmission mode, the second transmission mode transmits the monitoring data at a lower frequency, therefore is capable of satisfying a scenario with a lower requirement for human body monitoring, a scenario where a communication environment is not suitable for low-power data transmission, a scenario which pays much attention to power consumption, and the likes. Because the mobile monitoring device 100 provides a relatively low power consumption monitoring mode and/or transmission mode, which enables the mobile monitoring device 100 to have a smaller size than a device that only includes a high frequency monitoring mode and a high frequency transmission mode, making it easier for a user to wear and improving user experience.

Furthermore, the wearable mobile monitoring device 100 is also capable of acquiring monitoring data at a third acquisition frequency, wherein the third acquisition frequency is higher than the second acquisition frequency mentioned above. In addition, the wearable mobile monitoring device 100 may also include a third transmission mode in which mode the monitoring data is transmitted to a monitoring device which is separate from the human body via a wireless communicative connection. The third transmission mode is a "temporary" transmission mode to which the second transmission mode is switched, when a third preset condition is detected. At this time, "temporary" can be understood as: when a transmission duration after switching to the third transmission mode reaches a first preset time threshold and the first preset condition is not detected, switch back to the second transmission mode.

The reason for providing the third transmission mode is that under a normal situation, when the first preset condition is satisfied, the first transmission mode is required to transmit to the monitoring device the monitoring data acquired at the first acquisition frequency, when the second preset condition is satisfied, the second transmission mode is required to transmit to the monitoring device the monitoring data acquired at the second acquisition frequency. However, in some temporary and sudden situations, although the first preset condition is not satisfied at present, however such temporary situations require a monitoring mode with a higher monitoring frequency and/or a transmission mode with a higher transmission frequency to obtain more monitoring data, and such temporary situations can generally be resolved quickly and will not last too long. In such situations, it is possible to "temporarily" switch to a transmission mode which is capable of acquiring more monitoring data, and after a certain time period, switch back to the second transmission mode to transmit the monitoring data acquired at the second acquisition freq200uency. Such temporary switching can satisfy some temporary or sudden requirements of user, and because the second transmission mode is quickly switched back to, it is also beneficial for the mobile monitoring device 100 to reduce power consumption as much as possible. Based on such application scenarios, a third transmission mode is provided. Therefore, the frequency for transmitting monitoring data in the third transmission mode is higher than the frequency for transmitting monitoring data in the second transmission mode, and/or the third acquisition frequency is greater than the second acquisition frequency. A relationship between the frequency for transmitting monitoring data in the third transmission mode and the frequency for transmitting monitoring data in the first transmission mode, is not limited, and they may be equal or unequal. Similarly, a relationship between the third acquisition frequency and the first acquisition frequency may not be limited, and they may be equal or unequal.

An example of the third preset condition is described below. In one example, the third preset condition may include a user instruction for switching a mode. In this example, the second transmission mode needs to be switched to the third transmission mode, because a user instruction is received. For example, as a medical condition of someone patient has improved, the mobile monitoring device 100 uses the second transmission mode with a lower transmission frequency to transmit data to the monitoring device. At this time, while the medical staff make rounds and find that the patient is not in a ward, then the medical staff can transmit a mode switching instruction. Based on the instruction, the mobile monitoring device 100 can temporarily switch the second transmission mode to the third transmission mode, and transmit monitoring data in the third transmission mode for a certain time period, which time period can be preset or required by a user instruction, so as to satisfy a temporary viewing requirement of the medical staff. After the medical staff has viewed more monitoring data for a certain time period, they can ensure whether any unexpected change exists in the medical condition of the patient. If no additional instruction is received, it generally means that the medical staff have ensured that no unexpected change exists in the medical condition of the patient. Therefore, the mobile monitoring device 100 can be switched back to the second transmission mode after transmitting data in the third transmission mode for a certain time period, if the first preset condition is not detected.

For another example, as a medical condition of someone patient has improved, the mobile monitoring device 100 uses the second transmission mode with a lower transmission frequency to transmit data to the monitoring device. At this time, the medical staff finds that a certain risk exists in a trend of data through the monitoring data displayed by the monitoring device. At this time, the medical staff can transmit a mode switching instruction. Based on the instruction, the mobile monitoring device 100 can temporarily switch the second transmission mode to the third transmission mode, and transmit monitoring data in the third transmission mode for a certain time period, which time period can be preset or required by a user instruction, so as to satisfy a temporary viewing requirement of the medical staff. After the medical staff views more monitoring data for a certain time period, they can ensure whether any unexpected change exists in the medical condition of the patient. If no additional instruction is received, it generally means that the medical staff have ensured that no unexpected change exists in the medical condition of the patient. Therefore, the mobile monitoring device 100 can be switched back to the second transmission mode after transmitting data in the third transmission mode for a certain time period, if the first preset condition is not detected.

In the above two examples, upon receiving a user instruction, a temporary mode switching can be performed to satisfy a temporary viewing requirement of a user. In other examples, there may be other situations where a temporary mode switching is required, which are not given here. In general, such temporary mode switching enables the mobile monitoring device 100 to satisfy various complex scenarios while minimizing power consumption.

In an embodiment of this disclosure, the first preset condition mentioned above may include: an indicator, which reflects a communication state between the mobile monitoring device 100 and the monitoring device, satisfies a fourth preset condition, and/or an indicator, which reflects a health state of the human body, satisfies a fifth preset condition. In this embodiment, the first preset condition is mainly associated with at least one of: a communication state between the mobile monitoring device 100 and the monitoring device, and a health state of the human body. Generally, it is easy to understand that when the communication state between the mobile monitoring device 100 and the monitoring device is good, the communication between the mobile monitoring device 100 and the monitoring device does not consume too much power, the first transmission mode with a higher transmission frequency can be selected to obtain more monitoring data while saving power as much as possible. In addition, when the health state of the human body is not very good has a relatively high risk, the first transmission mode with a higher transmission frequency can be selected to ensure a monitoring safety of the patient based on more intensive monitoring data.

In an embodiment of this disclosure, an indicator, which reflects a communication state between the mobile monitoring device 100 and the monitoring device, satisfying a fourth preset condition, includes at least one of: a communicative connection between the mobile monitoring device 100 and the monitoring device is a point-to-point wireless communicative connection; when the monitoring device is a monitor located inside a ward, the mobile monitoring device 100 is also located inside said ward; a distance between the mobile monitoring device 100 and the monitoring device is less than a first preset distance threshold.

Wherein, the point-to-point wireless communicative connection may include a Bluetooth connection, a Wireless Fidelity (referred to as WIFI) direct connection, a Wireless Medical Telemetry Services (referred to as WMTS) direct connection, and the likes. Since the point-to-point communicative connection has lower power consumption, even if the transmission frequency is higher, there will not be much power consumption, so that the first transmission mode can be used in this case.

When the monitoring device is a monitor located inside a ward and the mobile monitoring device 100 is also located inside said ward, since both the mobile monitoring device 100 and the monitoring device are inside the same ward, the communication distance between them is short, and even if the transmission frequency is high, there will not be too much power consumption. Therefore, the first transmission mode can be used in this case. In addition, the mobile monitoring device 100 is located inside the ward, that is, the patient wearing the mobile monitoring device 100 has not left the ward, which may mean that the medical condition of the patient is not suitable for getting out of bed. At this time, it is more necessary to use the first transmission mode with a higher transmission frequency to ensure a monitoring safety.

The distance between the mobile monitoring device 100 and the monitoring device is less than the first preset distance threshold, which is similar to the situation where both the mobile monitoring device 100 and the monitoring device are inside the same ward. Since the distance between the mobile monitoring device 100 and the monitoring device is small and the communication distance is short, even if the transmission frequency is high, there will not be much power consumption. Therefore, the first transmission mode can be used in this case. In addition, the distance between the mobile monitoring device 100 and the monitoring device is small. If the monitoring device is a monitor inside a ward, it also means that the mobile monitoring device 100 is also located inside said ward, that is, the patient wearing the mobile monitoring device 100 has not walked out of the ward. This may mean that the medical condition of the patient is not suitable for getting out of bed. At this time, it is more necessary to use the first transmission mode with a higher transmission frequency to ensure a monitoring safety.

In an embodiment of this disclosure, an indicator, which reflects a health state of the human body, satisfying a fifth preset condition, includes at least one of: a monitoring time for the human body by the mobile monitoring device 100 does not reach a second preset time threshold; an early warning score (referred to as EWS) of the human body based on the monitoring data is lower than a first preset score threshold; at least one item of the monitoring data has an abnormality at a current moment; at least one item of the monitoring data has no abnormality at a current moment, but has an abnormality within a preset time before said current moment, and a duration of the preset time is a third preset time threshold; an accident event for movement occurs to the human body.

In this embodiment, at least one of the monitoring time, the EWS based on the monitoring data, the monitoring data itself, and the other accident event is used as an indicator which reflects the health state of the human body. Wherein, the monitoring time, for example, a time within 8 hours after surgery is a relatively important monitoring time. During this time, the patient needs to be closely monitored to ensure that a possible postoperative risk for the patient is monitored. Therefore, when the monitoring time of the patient by the mobile monitoring device is less than 8 hours, the first transmission mode with a higher transmission frequency should be used. Of course, this is merely exemplary, and in other cases, other time thresholds (collectively referred to as a second preset time threshold) may be used for determination.

In addition, the EWS based on monitoring data can reflect an overall medical condition of the patient. When the EWS is lower than the first preset score threshold, it can be considered that overall medical condition of the patient still requires more monitoring data to ensure safety. Therefore, the first transmission mode with a higher transmission frequency should be used at this time.

In addition, some important monitoring data (such as heart rate, blood oxygen saturation, etc.) may reflect a change in the medical condition and should be monitored immediately if any abnormity exists in said data, the first transmission mode with a higher transmission frequency should also be used for this case.

In addition, for some monitoring data, even if its abnormality disappears after a time period of monitoring or treatment and said data begins to no longer be abnormal, close monitoring can be continued for a time period to ensure that the situation without such abnormality is maintained for a time period before temporarily considering that the medical condition is stable and no longer requires close monitoring. This can further improve the monitoring safety. In this case, that is, even if the monitoring data has no abnormality at a current moment, it is still necessary to use the first transmission mode with a higher transmission frequency if an abnormality exists within a preset time before the current moment. That is, after the abnormality disappears, the transmission mode is not switched immediately, data is continuously transmitted in the first transmission mode with a higher transmission frequency within a preset time, and then the transmission mode switched to the second transmission mode after the preset time is over.

In addition, when a patient accident event for movement occurs, such as a patient falling, the patient may fall because of an abnormality, or an abnormality may occur due to the falling. Therefore, the first transmission mode with a higher transmission frequency should be used at this time to ensure a monitoring safety. After the monitoring data is transmitted in the first transmission mode for a time period, if no abnormality is detected or if an abnormality existed but has disappeared, the second transmission mode can be switched back to.

In an embodiment of this disclosure, the aforementioned second preset condition may include: under the premise that no user instruction for switching a transmission mode is detected, an indicator, which reflects a communication state between the mobile monitoring device 100 and the monitoring device, satisfies a sixth preset condition, and/or, under the premise that no user instruction for switching a transmission mode is detected, an indicator, which reflects a health state of the human body, satisfies a seventh preset condition. In this embodiment, the second preset condition is mainly associated with at least one of: a communication state between the mobile monitoring device 100 and the monitoring device, and a health state of the human body. Generally, it is easy to understand that when the communication state between the mobile monitoring device 100 and the monitoring device is not suitable for high-frequency data transmission, or when high-frequency data transmission consumes a lot of power, a second transmission mode with a lower transmission frequency can be selected to save power consumption. In addition, when the human body is in good health or the risk is small, a second transmission mode with a lower transmission frequency can be selected to save power consumption.

In an embodiment of this disclosure, an indicator, which reflects a communication state between the mobile monitoring device 100 and the monitoring device, satisfying a sixth preset condition, includes at least one of: a communicative connection between the mobile monitoring device 100 and the monitoring device is not a point-to-point wireless communicative connection; when the monitoring device is a monitor located inside a ward, the mobile monitoring device 100 is located outside said ward; a distance between the mobile monitoring device 100 and the monitoring device is not less than a second preset distance threshold.

The connection which is not a point-to-point wireless communicative connection may include a wireless communicative connection via an access point (AP) network connection. Since not point-to-point communicative connection consume a lot of power, in this case a second transmission mode with a lower transmission frequency may be used to save power consumption.

When the monitoring device is a monitor located inside a ward and the mobile monitoring device 100 is located outside said ward, the communication distance between them is far and a high transmission frequency will consume too much power. Therefore, in this case, a second transmission mode with a lower transmission frequency can be used to save power consumption. In addition, the mobile monitoring device 100 is located outside said ward, that is, the patient wearing the mobile monitoring device 100 has walked out of the ward, which may mean that the medical condition of the patient has improved. At this time, there is no need to use the first transmission mode with a higher transmission frequency, and the second transmission mode with a lower transmission frequency can be used.

The distance between the mobile monitoring device 100 and the monitoring device is not less than the second preset distance threshold, which is similar to the situation where the mobile monitoring device 100 and the monitoring device are not inside the same ward. Due to the large distance and the long communication distance between the mobile monitoring device 100 and the monitoring device, a high transmission frequency will consume too much power. Therefore, in this case, a second transmission mode with a lower transmission frequency can be used to save power consumption. In addition, the mobile monitoring device 100 is far away from the monitoring device. If the monitoring device is a monitor inside the ward, it also means that the mobile monitoring device 100 may be located outside said ward, that is, the patient wearing the mobile monitoring device 100 has walked out of the ward, which may mean that the medical condition of the patient has improved. At this time, there is no need to use the first transmission mode with a higher transmission frequency, and the second transmission mode with a lower transmission frequency can be used.

In an embodiment of this disclosure, an indicator, which reflects a health state of the human body, satisfying a seventh preset condition, includes at least one of: a monitoring time for the human body by the mobile monitoring device 100 reaches a fourth preset time threshold; an early warning score (referred to as EWS) of the human body based on the monitoring data is not lower than a second preset score threshold; the monitoring data has no abnormality; and no accident event for movement occurs to the human body.

In this embodiment, at least one of the monitoring time, the EWS based on the monitoring data, the monitoring data itself, and the other accident event is used as an indicator which reflects the health state of the human body. Wherein, the monitoring time, for example, a time within 8 hours after surgery is a relatively important monitoring time. During this time, the patient needs to be closely monitored to ensure that a possible postoperative risk for the patient is monitored. After this time, the patient condition is generally stable. Therefore, after the monitoring time of the patient by the mobile monitoring device reaches 8 hours, the second transmission mode with a lower transmission frequency can be used. Of course, this is merely exemplary, and in other cases, other time thresholds (collectively referred to as a fourth preset time threshold) may be used for determination.

In addition, the EWS based on the monitoring data can reflect an overall medical condition of the patient. When the EWS is not lower than the second preset score threshold, the overall medical condition of the patient can be considered stable or good. Therefore, the second transmission mode with a lower transmission frequency should be used at this time.

In addition, no abnormality existing in the monitoring data generally indicates that the overall medical condition of the patient is stable or good, so that the second transmission mode with a lower transmission frequency should be used at this time. Similarly, if the patient does not have any accident event for movement, there is no need to use the first transmission mode with a higher transmission frequency, and the second transmission mode with a lower transmission frequency can be used.

In an embodiment of this disclosure, the processor 120 can also be configured to: output countdown indication information according to the first preset time threshold mentioned above to indicate to a user that the third transmission mode is about to be switched back to the second transmission mode; wherein a start moment of countdown in the countdown indication information is when the second transmission mode is switched to the third transmission mode.

According to the foregoing, the first preset time threshold is a duration for transmitting the monitoring data in the third transmission mode after switching from the second transmission mode to the third transmission mode. Based on this, in this embodiment, countdown indication information is provided according to the first preset time threshold. Specifically, when the second transmission mode is switched to the third transmission mode, a countdown, for example, 120 seconds or other suitable time, can be started, and the third transmission mode is switched back to the second transmission mode at the end of the countdown. Based on the countdown indication information, the user can intuitively understand the duration of the third transmission mode, and determine whether the current situation is really suitable for switching back to the second transmission mode when the duration ends. For example, if the user finds that monitoring for a further time period is still required when the countdown is about to end, an instruction can be given to continue the third transmission mode; or, when the third transmission mode is switched back to the second transmission mode after the countdown ends, the user can give an instruction again to switch back to the third transmission mode; or, if the first preset condition is detected before the countdown ends, the third transmission mode can be switched back to the first transmission mode. In general, based on the countdown indication information, the user can be reminded of the switching in the transmission mode that will occur later, this is convenient for the user to confirm whether such a switching is appropriate, and to determine an action to be taken afterward (for example, after switching back to the second transmission mode, due to the lower transmission frequency, the user may need to pay attention to some emergencies that cannot be reflected in the data in time, etc.).

In an embodiment of this disclosure, the processor 120 can also be configured to: output countdown indication information according to a third preset time threshold mentioned above to indicate to a user that the first transmission mode is about to be switched to the second transmission mode; wherein a start moment of countdown in the countdown indication information is when the abnormality disappears.

According to the foregoing, the third preset time threshold is a duration in which the monitoring data is transmitted by continuing to use the first transmission mode, after the abnormality in the monitoring data disappears. Based on this, in this embodiment, countdown indication information is provided according to the third preset time threshold, specifically, a countdown, for example, 120 seconds or other suitable time, is started, after the abnormality in the monitoring data disappears, and the first transmission mode is switched back to the second transmission mode at an end of the countdown. Based on the countdown indication information, the user can intuitively understand that the abnormality has disappeared, the first transmission mode will be switched back to the second transmission mode with a lower transmission frequency, and an action should be taken afterward, for example, after switching back to the second transmission mode, due to the lower transmission frequency, the user may need to pay attention to some emergencies that cannot be reflected in the data in a timely manner, etc.

In an embodiment of this disclosure, the countdown indication information described above can be displayed on a display of the mobile monitoring device 100 and/or a display of the monitoring device, and/or played through a speaker of the mobile monitoring device 100 and/or a speaker of the monitoring device.

In an embodiment of this disclosure, the monitoring device mentioned above may be a monitor (e.g., located inside a ward) or a central monitoring system (e.g., located at a nurse station).

In an embodiment of this disclosure, the first transmission mode mentioned above may include a continuous transmission mode, the second transmission mode mentioned above may include an intermittent transmission mode, and the third transmission mode mentioned above may include a temporary continuous transmission mode, wherein: the continuous transmission mode refers to transmitting data in a first preset number of continuous transmission cycles; the intermittent transmission mode refers to transmitting data only in a first part of transmission cycles among a continuous preset of transmission cycles; the temporary continuous transmission mode refers to transmitting data only in a second part of transmission cycles among a continuous preset of transmission cycles, wherein a number of the second part of transmission cycles is greater than a number of the first part of transmission cycles.

In this embodiment, the first transmission mode includes a continuous transmission mode, and the second transmission mode includes an intermittent transmission mode. It is easy to understand that the frequency for transmitting monitoring data in the continuous transmission mode is higher than that in the intermittent transmission mode. In addition, the third transmission mode includes a temporary continuous transmission mode, wherein the temporary continuous transmission mode is a "temporary" continuous transmission mode, which can be specifically reflected as follows: compared with the continuous transmission mode, the temporary continuous transmission mode also transmits data in a preset number of continuous transmission cycles, but said transmission cycles are less than the transmission cycles in the continuous transmission mode; or, compared with the intermittent transmission mode, the temporary continuous transmission mode also transmits data in some cycles in the preset number of continuous transmission cycles, and said some cycles may be continuous, and a number of said some cycles is greater than a number of cycle(s) for transmitting data in the intermittent transmission mode. The continuous transmission mode, the intermittent transmission mode, and the temporary continuous transmission mode can be respectively adapted to the scenarios corresponding to the first transmission mode, the second transmission mode, and the third transmission mode described above, and will not be described in detail here.

In an embodiment of this disclosure, the monitoring data transmitted in the continuous transmission mode includes real-time monitoring data; and the monitoring data transmitted in the temporary continuous transmission mode includes an analysis result of real-time monitoring data. In this embodiment, respective monitoring data transmitted in the continuous transmission mode and the temporary continuous transmission mode may be different. In the continuous transmission mode, the mobile monitoring device 100 transmits real-time monitoring data to the monitoring device, and the monitoring device can display such real-time data and execute a more complex algorithm to analyze the real-time data to obtain some analysis result data. In the temporary continuous transmission mode, after the mobile monitoring device 100 obtains real-time monitoring data, since the mobile monitoring device 100 does not need to transmit the real-time monitoring data in real time, the mobile monitoring device 100 can analyze the real-time monitoring data and transmit analysis result data to the monitoring device. The monitoring device can directly display such analysis result data without having to perform complex algorithms itself or just need to perform some algorithms that are not performed on the mobile monitoring device 100.

The above exemplary shows a wearable mobile monitoring device 100 according to an embodiment of this disclosure. Based on the above description, after acquiring the monitoring data of the human body, the wearable mobile monitoring device 100 according to the embodiment of this disclosure can transmit the monitoring data to a monitoring device which is separate from the human body through different transmission modes. These different transmission modes can satisfy a scenario with a high monitoring requirement, a scenario with a low power consumption requirement, and a scenario that requires temporary mode switching, so as to satisfy various complex scenarios while minimizing power consumption.

The following describes a monitoring system provided according to another aspect of this disclosure.

FIG. 2 is a structural block diagram of a monitoring system 200 according to an embodiment of this disclosure. As shown in FIG. 2, the monitoring system 20 may include a wearable mobile monitoring device 100 worn on a human body and a monitoring device 200 which is separate from the human body. The mobile monitoring device is configured to detect monitoring data of a human body; the monitoring device 200 is configured to obtain and display the monitoring data; the mobile monitoring device is further configured to perform following operations: transmitting the monitoring data to the monitoring device 200 through a communicative connection which is established between the mobile monitoring device 100 and the monitoring device 200, wherein transmitting the monitoring data to the monitoring device 200 includes: under a first preset condition, transmitting, in a first transmission mode, the monitoring data which is acquired at a first acquisition frequency; under a second preset condition, transmitting, in a second transmission mode, the monitoring data which is acquired at a second acquisition frequency; wherein a frequency for transmitting the monitoring data in the first transmission mode is higher than a frequency for transmitting the monitoring data in the second transmission mode, and/or the first acquisition frequency is higher than the second acquisition frequency; when the monitoring data is transmitted in the second transmission mode; if a third preset condition is detected, switching to a third transmission mode in which mode the monitoring data, which is acquired at a third acquisition frequency, is transmitted, and further switching back to the second transmission mode, when a transmission duration after switching to the third transmission mode reaches a first preset time threshold and the first preset condition is not detected; wherein a frequency for transmitting the monitoring data in the third transmission mode is higher than the frequency for transmitting the monitoring data in the second transmission mode, and/or the third acquisition frequency is higher than the second acquisition frequency.

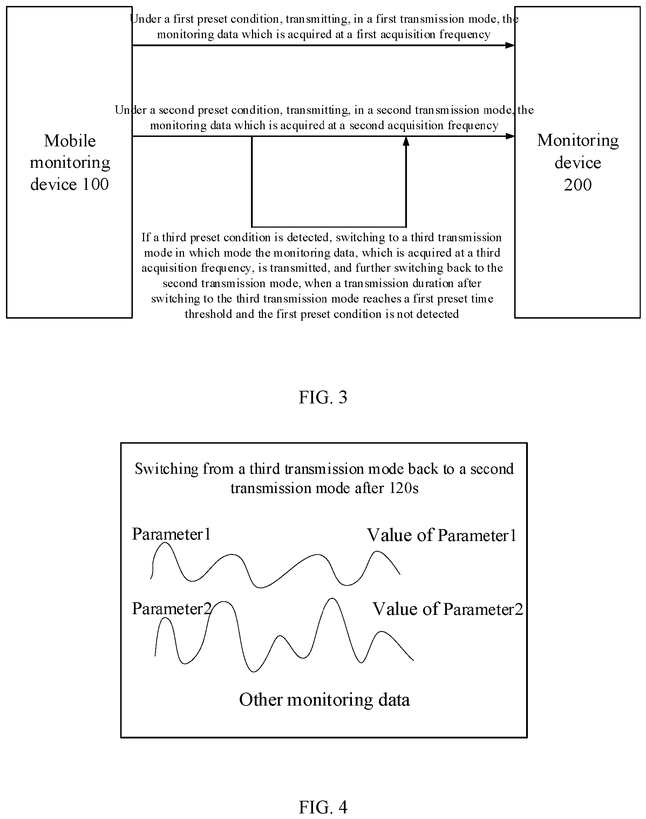

In an embodiment of this disclosure, after acquiring monitoring data, the wearable mobile monitoring device 100 in the monitoring system 20 transmits the monitoring data to the monitoring device 200 which is separate from the human body through a wireless communicative connection. In different situations, the mobile monitoring device 100 may acquire monitoring data at different acquisition frequencies, and may also transmit the monitoring data in different transmission modes. For example, under the first preset condition, the mobile monitoring device 100 transmits the monitoring data acquired at the first acquisition frequency in the first transmission mode; under the second preset condition, the mobile monitoring device 100 transmits the monitoring data acquired at the second acquisition frequency in the second transmission mode, as shown in FIG. 3. The frequency for transmitting the monitoring data in the first transmission mode is higher than the frequency for transmitting the monitoring data in the second transmission mode, and/or the first acquisition frequency is higher than the second acquisition frequency. Therefore, in the monitoring system 20 according to an embodiment of this disclosure, the mobile monitoring device 100 can acquire monitoring data at different acquisition frequencies, and can also use different transmission modes to transmit monitoring data to the monitoring device 200, so as to satisfy the transmission requirements under different conditions. Specifically, compared with the second acquisition frequency, the first acquisition frequency is higher, therefore is capable of satisfying a scenario with a higher requirement for human body monitoring, a scenario which does not pay much attention to power consumption, and the likes. Similarly, compared with the first acquisition frequency, the second acquisition frequency is lower, therefore is capable of satisfying a scenario with a lower requirement for human body monitoring, a scenario which pays more attention to power consumption, and the likes. Similarly, compared with the second transmission mode, the first transmission mode transmits the monitoring data at a higher frequency, therefore is capable of satisfying a scenario with a higher requirement for human body monitoring, a scenario where a communication environment is suitable for low-power data transmission, a scenario which does not pay much attention to power consumption, and the likes. Compared with the first transmission mode, the second transmission mode transmits the monitoring data at a lower frequency, therefore is capable of satisfying a scenario with a lower requirement for human body monitoring, a scenario where a communication environment is not suitable for low-power data transmission, a scenario which pays much attention to power consumption, and the likes. Because the mobile monitoring device 100 provides a relatively low power consumption monitoring mode and/or transmission mode, which enables the mobile monitoring device 100 to have a smaller size than a device that only includes a high frequency monitoring mode and a high frequency transmission mode, making it easier for a user to wear and improving user experience.

Furthermore, the wearable mobile monitoring device 100 in the monitoring system 20 is also capable of acquiring monitoring data at a third acquisition frequency, wherein the third acquisition frequency is higher than the second acquisition frequency mentioned above. In addition, the wearable mobile monitoring device 100 may also include a third transmission mode in which mode the monitoring data is transmitted to the monitoring device 200 which is separate from the human body through a wireless communicative connection. The third transmission mode is a "temporary" transmission mode to which the second transmission mode is switched when a third preset condition is detected. At this time, "temporary" can be understood as: when a transmission duration after switching to the third transmission mode reaches a first preset time threshold and the first preset condition is not detected, switch back to the second transmission mode, as shown in FIG. 3.

The reason for providing the third transmission mode is that under a normal situation, when the first preset condition is satisfied, the first transmission mode is required to transmit to the monitoring device 200 the monitoring data acquired at the first acquisition frequency, when the second preset condition is satisfied, the second transmission mode is required to transmit to the monitoring device 200 the monitoring data acquired at the second acquisition frequency. However, in some temporary and sudden situations, although the first preset condition is not satisfied at present, however such temporary situations require a monitoring mode with a higher monitoring frequency and/or a transmission mode with a higher transmission frequency to obtain more monitoring data, and such temporary situations can generally be resolved quickly and will not last too long. In such situations, it is possible to "temporarily" switch to a transmission mode which is capable of acquiring more monitoring data, and after a certain time period, switch back to the second transmission mode to transmit the monitoring data acquired at the second acquisition frequency. Such temporary switching can satisfy some temporary or sudden requirements of user, and because the second transmission mode is quickly switched back to, it is also beneficial for the mobile monitoring device 100 to reduce power consumption as much as possible. Based on such application scenarios, a third transmission mode is provided. Therefore, the frequency for transmitting monitoring data in the third transmission mode is higher than the frequency for transmitting monitoring data in the second transmission mode, and/or the third acquisition frequency is greater than the second acquisition frequency. A relationship between the frequency for transmitting monitoring data in the third transmission mode and the frequency for transmitting monitoring data in the first transmission mode, is not limited, and they may be equal or unequal. Similarly, a relationship between the third acquisition frequency and the first acquisition frequency may not be limited, and they may be equal or unequal.

An example of the third preset condition is described below. In one example, the third preset condition may include a user instruction for switching a mode. In this example, the second transmission mode needs to be switched to the third transmission mode, because a user instruction is received. For example, as a medical condition of someone patient has improved, the mobile monitoring device 100 uses the second transmission mode with a lower transmission frequency to transmit data to the monitoring device 200. At this time, while the medical staff make rounds and find that the patient is not in a ward, then the medical staff can transmit a mode switching instruction. Based on the instruction, the mobile monitoring device 100 can temporarily switch the second transmission mode to the third transmission mode, and transmit monitoring data in the third transmission mode for a certain time period, which time period can be preset or required by a user instruction, so as to satisfy a temporary viewing requirement of the medical staff. After the medical staff has viewed more monitoring data for a certain time period, they can ensure whether any unexpected change exists in the medical condition of the patient. If no additional instruction is received, it generally means that the medical staff have ensured that no unexpected change exists in the medical condition of the patient. Therefore, the mobile monitoring device 100 can be switched back to the second transmission mode after transmitting data in the third transmission mode for a certain time period, if the first preset condition is not detected.

For another example, as a medical condition of someone patient has improved, the mobile monitoring device 100 uses the second transmission mode with a lower transmission frequency to transmit data to the monitoring device 200. At this time, the medical staff finds that a certain risk exists in a trend of data through the monitoring data displayed by the monitoring device 200. At this time, the medical staff can transmit a mode switching instruction. Based on the instruction, the mobile monitoring device 100 can temporarily switch the second transmission mode to the third transmission mode, and transmit monitoring data in the third transmission mode for a certain time period, which time period can be preset or required by a user instruction, so as to satisfy a temporary viewing requirement of the medical staff. After the medical staff views more monitoring data for a certain time period, they can ensure whether any unexpected change exists in the medical condition of the patient. If no additional instruction is received, it generally means that the medical staff have ensured that no unexpected change exists in the medical condition of the patient. Therefore, the mobile monitoring device 100 can be switched back to the second transmission mode after transmitting data in the third transmission mode for a certain time period, if the first preset condition is not detected.

In the above two examples, upon receiving a user instruction, a temporary mode switching can be performed to satisfy a temporary viewing requirement of a user. In other examples, there may be other situations where a temporary mode switching is required, which are not given here. In general, such temporary mode switching enables the mobile monitoring device 100 to satisfy various complex scenarios while minimizing power consumption.

In an embodiment of this disclosure, the first preset condition mentioned above may include: an indicator, which reflects a communication state between the mobile monitoring device 100 and the monitoring device 200, satisfies a fourth preset condition, and/or an indicator, which reflects a health state of the human body, satisfies a fifth preset condition. In this embodiment, the first preset condition is mainly associated with at least one of: a communication state between the mobile monitoring device 100 and the monitoring device 200, and a health state of the human body. Generally, it is easy to understand that when the communication state between the mobile monitoring device 100 and the monitoring device 200 is good, the communication between the mobile monitoring device 100 and the monitoring device does not consume too much power, the first transmission mode with a higher transmission frequency can be selected to obtain more monitoring data while saving power as much as possible. In addition, when the health state of the human body is not very good has a relatively high risk, the first transmission mode with a higher transmission frequency can be selected to ensure a monitoring safety of the patient based on more intensive monitoring data.

In an embodiment of this disclosure, an indicator, which reflects a communication state between the mobile monitoring device 100 and the monitoring device 200, satisfying a fourth preset condition, includes at least one of: a communicative connection between the mobile monitoring device 100 and the monitoring device 200 is a point-to-point wireless communicative connection; when the monitoring device 200 is a monitor located inside a ward, the mobile monitoring device 100 is also located inside said ward; a distance between the mobile monitoring device 100 and the monitoring device 200 is less than a first preset distance threshold.

Wherein, the point-to-point wireless communicative connection may include a Bluetooth connection, a Wireless Fidelity (referred to as WIFI) direct connection, a Wireless Medical Telemetry Services (referred to as WMTS) direct connection, and the likes. Since the point-to-point communicative connection has lower power consumption, even if the transmission frequency is higher, there will not be much power consumption, so that the first transmission mode can be used in this case.

When the monitoring device 200 is a monitor located inside a ward and the mobile monitoring device 100 is also located inside said ward, since both the mobile monitoring device 100 and the monitoring device 200 are inside the same ward, the communication distance between them is short, and even if the transmission frequency is high, there will not be too much power consumption. Therefore, the first transmission mode can be used in this case. In addition, the mobile monitoring device 100 is located inside the ward, that is, the patient wearing the mobile monitoring device 100 has not left the ward, which may mean that the medical condition of the patient is not suitable for getting out of bed. At this time, it is more necessary to use the first transmission mode with a higher transmission frequency to ensure a monitoring safety.

The distance between the mobile monitoring device 100 and the monitoring device 200 is less than the first preset distance threshold, which is similar to the situation where both the mobile monitoring device 100 and the monitoring device 200 are inside the same ward. Since the distance between the mobile monitoring device 100 and the monitoring device 200 is small and the communication distance is short, even if the transmission frequency is high, there will not be much power consumption. Therefore, the first transmission mode can be used in this case. In addition, the distance between the mobile monitoring device 100 and the monitoring device 200 is small. If the monitoring device 200 is a monitor inside a ward, it also means that the mobile monitoring device 100 is also located inside said ward, that is, the patient wearing the mobile monitoring device 100 has not walked out of the ward. This may mean that the medical condition of the patient is not suitable for getting out of bed. At this time, it is more necessary to use the first transmission mode with a higher transmission frequency to ensure a monitoring safety.

In an embodiment of this disclosure, an indicator, which reflects a health state of the human body, satisfying a fifth preset condition, includes at least one of: a monitoring time for the human body by the mobile monitoring device 100 does not reach a second preset time threshold; an early warning score (referred to as EWS) of the human body based on the monitoring data is lower than a first preset score threshold; at least one item of the monitoring data has an abnormality at a current moment; at least one item of the monitoring data has no abnormality at a current moment, but has an abnormality within a preset time before said current moment, and a duration of the preset time is a third preset time threshold; an accident event for movement occurs to the human body.

In this embodiment, at least one of the monitoring time, the EWS based on the monitoring data, the monitoring data itself, and the other accident event is used as an indicator which reflects the health state of the human body. Wherein, the monitoring time, for example, a time within 8 hours after surgery is a relatively important monitoring time. During this time, the patient needs to be closely monitored to ensure that a possible postoperative risk for the patient is monitored. Therefore, when the monitoring time of the patient by the mobile monitoring device is less than 8 hours, the first transmission mode with a higher transmission frequency should be used. Of course, this is merely exemplary, and in other cases, other time thresholds (collectively referred to as a second preset time threshold) may be used for determination.

In addition, the EWS based on monitoring data can reflect an overall medical condition of the patient. When the EWS is lower than the first preset score threshold, it can be considered that overall medical condition of the patient still requires more monitoring data to ensure safety. Therefore, the first transmission mode with a higher transmission frequency should be used at this time.

In addition, some important monitoring data (such as heart rate, blood oxygen saturation, etc.) may reflect a change in the medical condition and should be monitored immediately if any abnormity exists in said data, the first transmission mode with a higher transmission frequency should also be used for this case.

In addition, for some monitoring data, even if its abnormality disappears after a time period of monitoring or treatment and said data begins to no longer be abnormal, close monitoring can be continued for a time period to ensure that the situation without such abnormality is maintained for a time period before temporarily considering that the medical condition is stable and no longer requires close monitoring. This can further improve the monitoring safety. In this case, that is, even if the monitoring data has no abnormality at a current moment, it is still necessary to use the first transmission mode with a higher transmission frequency if an abnormality exists within a preset time before the current moment. That is, after the abnormality disappears, the transmission mode is not switched immediately, data is continuously transmitted in the first transmission mode with a higher transmission frequency within a preset time, and then the transmission mode switched to the second transmission mode after the preset time is over.

In addition, when a patient accident event for movement occurs, such as a patient falling, the patient may fall because of an abnormality, or an abnormality may occur due to the falling. Therefore, the first transmission mode with a higher transmission frequency should be used at this time to ensure a monitoring safety. After the monitoring data is transmitted in the first transmission mode for a time period, if no abnormality is detected or if an abnormality existed but has disappeared, the second transmission mode can be switched back to.

In an embodiment of this disclosure, the aforementioned second preset condition may include: under the premise that no user instruction for switching a transmission mode is detected, an indicator, which reflects a communication state between the mobile monitoring device 100 and the monitoring device 200, satisfies a sixth preset condition, and/or, under the premise that no user instruction for switching a transmission mode is detected, an indicator, which reflects a health state of the human body, satisfies a seventh preset condition. In this embodiment, the second preset condition is mainly associated with at least one of: a communication state between the mobile monitoring device 100 and the monitoring device 200, and a health state of the human body. Generally, it is easy to understand that when the communication state between the mobile monitoring device 100 and the monitoring device 200 is not suitable for high-frequency data transmission, or when high-frequency data transmission consumes a lot of power, a second transmission mode with a lower transmission frequency can be selected to save power consumption. In addition, when the human body is in good health or the risk is small, a second transmission mode with a lower transmission frequency can be selected to save power consumption.

In an embodiment of this disclosure, an indicator, which reflects a communication state between the mobile monitoring device 100 and the monitoring device 200, satisfying a sixth preset condition, includes at least one of: a communicative connection between the mobile monitoring device 100 and the monitoring device 200 is not a point-to-point wireless communicative connection; when the monitoring device 200 is a monitor located inside a ward, the mobile monitoring device 100 is located outside said ward; a distance between the mobile monitoring device 100 and the monitoring device 200 is not less than a second preset distance threshold.

The connection, which is not a point-to-point wireless communicative connection, may include a wireless communicative connection via an access point (AP) network connection. Since not point-to-point communicative connection consume a lot of power, in this case a second transmission mode with a lower transmission frequency may be used to save power consumption.

When the monitoring device 200 is a monitor located inside a ward and the mobile monitoring device 100 is located outside said ward, the communication distance between them is far and a high transmission frequency will consume too much power. Therefore, in this case, a second transmission mode with a lower transmission frequency can be used to save power consumption. In addition, the mobile monitoring device 100 is located outside said ward, that is, the patient wearing the mobile monitoring device 100 has walked out of the ward, which may mean that the medical condition of the patient has improved. At this time, there is no need to use the first transmission mode with a higher transmission frequency, and the second transmission mode with a lower transmission frequency can be used.

The distance between the mobile monitoring device 100 and the monitoring device 200 is not less than the second preset distance threshold, which is similar to the situation where the mobile monitoring device 100 and the monitoring device 200 are not inside the same ward. Due to the large distance and the long communication distance between the mobile monitoring device 100 and the monitoring device 200, a high transmission frequency will consume too much power. Therefore, in this case, a second transmission mode with a lower transmission frequency can be used to save power consumption. In addition, the mobile monitoring device 100 is far separate from the monitoring device 200. If the monitoring device 200 is a monitor inside the ward, it also means that the mobile monitoring device 100 may be located outside said ward, that is, the patient wearing the mobile monitoring device 100 has walked out of the ward, which may mean that the medical condition of the patient has improved. At this time, there is no need to use the first transmission mode with a higher transmission frequency, and the second transmission mode with a lower transmission frequency can be used.

In an embodiment of this disclosure, an indicator, which reflects a health state of the human body, satisfying a seventh preset condition, includes at least one of: a monitoring time for the human body by the mobile monitoring device 100 reaches a fourth preset time threshold; an early warning score (referred to as EWS) of the human body based on the monitoring data is not lower than a second preset score threshold; the monitoring data has no abnormality; and no accident event for movement occurs to the human body.

In this embodiment, at least one of the monitoring time, the EWS based on the monitoring data, the monitoring data itself, and the other accident event is used as an indicator which reflects the health state of the human body. Wherein, the monitoring time, for example, a time within 8 hours after surgery is a relatively important monitoring time. During this time, the patient needs to be closely monitored to ensure that a possible postoperative risk for the patient is monitored. After this time, the patient condition is generally stable. Therefore, after the monitoring time of the patient by the mobile monitoring device reaches 8 hours, the second transmission mode with a lower transmission frequency can be used. Of course, this is merely exemplary, and in other cases, other time thresholds (collectively referred to as a fourth preset time threshold) may be used for determination.

In addition, the EWS based on the monitoring data can reflect an overall medical condition of the patient. When the EWS is not lower than the second preset score threshold, the overall medical condition of the patient can be considered stable or good. Therefore, the second transmission mode with a lower transmission frequency should be used at this time.

In addition, no abnormality existing in the monitoring data generally indicates that the overall medical condition of the patient is stable or good, so that the second transmission mode with a lower transmission frequency should be used at this time. Similarly, if the patient does not have any accident event for movement, there is no need to use the first transmission mode with a higher transmission frequency, and the second transmission mode with a lower transmission frequency can be used.

In an embodiment of this disclosure, the mobile monitoring device 100 can also be configured to: output countdown indication information according to the first preset time threshold mentioned above to indicate to a user that the third transmission mode is about to be switched back to the second transmission mode; wherein a start moment of countdown in the countdown indication information is when the second transmission mode is switched to the third transmission mode. Exemplarily, the countdown indication information may be displayed on a display of the mobile monitoring device 100 and/or a display of the monitoring device 200, and/or played through a speaker of the mobile monitoring device 100 and/or a speaker of the monitoring device 200. FIG. 4 is a diagram showing countdown indication information displayed on a display of the monitoring device 200. In this example, the countdown time is 120 seconds, which is merely exemplary. The values and waveforms of parameters 1 and 2 and other monitoring parameters are also displayed, which are all exemplary.

According to the foregoing, the first preset time threshold is a duration for transmitting the monitoring data in the third transmission mode after switching from the second transmission mode to the third transmission mode. Based on this, in this embodiment, countdown indication information is provided according to the first preset time threshold. Specifically, when the second transmission mode is switched to the third transmission mode, a countdown, for example, 120 seconds or other suitable time, can be started, and the third transmission mode is switched back to the second transmission mode at the end of the countdown . Based on the countdown indication information, the user can intuitively understand the duration of the third transmission mode, and determine whether the current situation is really suitable for switching back to the second transmission mode when the duration ends. For example, if the user finds that monitoring for a further time period is still required when the countdown is about to end, an instruction can be given to continue the third transmission mode; or, when the third transmission mode is switched back to the second transmission mode after the countdown ends, the user can give an instruction again to switch back to the third transmission mode; or, if the first preset condition is detected before the countdown ends, the third transmission mode can be switched back to the first transmission mode. In general, based on the countdown indication information, the user can be reminded of the switching in the transmission mode that will occur later, this is convenient for the user to confirm whether such a switching is appropriate, and to determine an action to be taken afterward (for example, after switching back to the second transmission mode, due to the lower transmission frequency, the user may need to pay attention to some emergencies that cannot be reflected in the data in time, etc.).

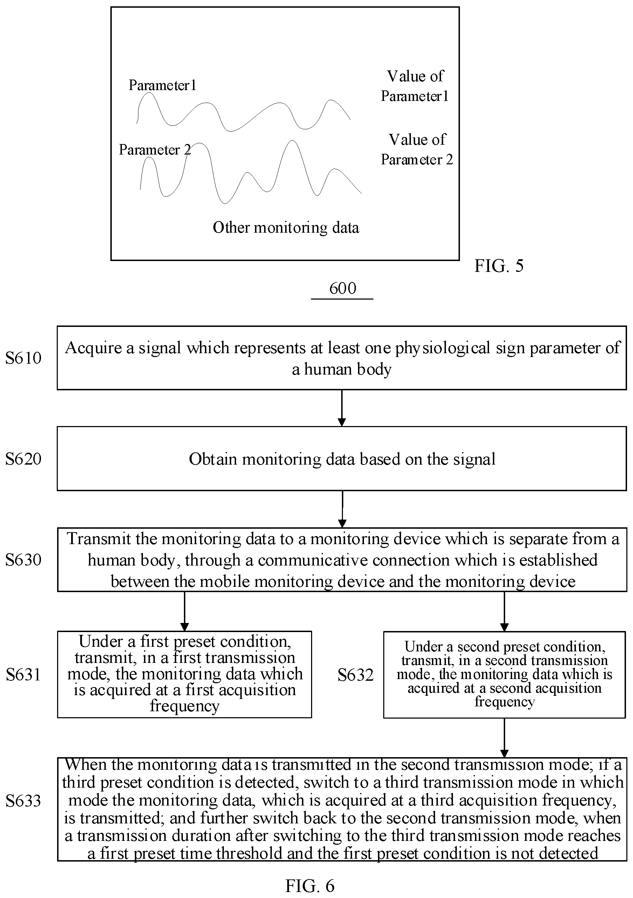

In an embodiment of this disclosure, the mobile monitoring device 100 can also be configured to: output countdown indication information according to a third preset time threshold mentioned above to indicate to a user that the first transmission mode is about to be switched to the second transmission mode; wherein a start moment of countdown in the countdown indication information is when the abnormality disappears. Exemplarily, the countdown indication information may be displayed on a display of the mobile monitoring device 100 and/or a display of the monitoring device 200, and/or played through a speaker of the mobile monitoring device 100 and/or a speaker of the monitoring device 200. FIG. 5 is a diagram showing countdown indication information displayed on a display of the monitoring device 200. In this example, the countdown time is 120 seconds, which is merely exemplary. The values and waveforms of parameters 1 and 2 and other monitoring parameters are also displayed, which are all exemplary.

According to the foregoing, the third preset time threshold is a duration in which the monitoring data is transmitted by continuing to use the first transmission mode, after the abnormality in the monitoring data disappears. Based on this, in this embodiment, countdown indication information is provided according to the third preset time threshold, specifically, a countdown, for example, 120 seconds or other suitable time, is started, after the abnormality in the monitoring data disappears, and the first transmission mode is switched back to the second transmission mode at an end of the countdown. Based on the countdown indication information, the user can intuitively understand that the abnormality has disappeared, the first transmission mode will be switched back to the second transmission mode with a lower transmission frequency, and an action should be taken afterward, for example, after switching back to the second transmission mode, due to the lower transmission frequency, the user may need to pay attention to some emergencies that cannot be reflected in the data in a timely manner, etc.

In an embodiment of this disclosure, the monitoring device 200 mentioned above may be a monitor (e.g., located inside a ward) or a central monitoring system 20 (e.g., located at a nurse station).

In an embodiment of this disclosure, the first transmission mode mentioned above may include a continuous transmission mode, the second transmission mode mentioned above may include an intermittent transmission mode, and the third transmission mode mentioned above may include a temporary continuous transmission mode, wherein: the continuous transmission mode refers to transmitting data in a preset number of continuous transmission cycles; the intermittent transmission mode refers to transmitting data only in a first part of transmission cycles among a continuous preset of transmission cycles; the temporary continuous transmission mode refers to transmitting data only in a second part of transmission cycles among a continuous preset of transmission cycles, wherein a number of the second part of transmission cycles is greater than a number of the first part of transmission cycles.

In this embodiment, the first transmission mode includes a continuous transmission mode, and the second transmission mode includes an intermittent transmission mode. It is easy to understand that the frequency for transmitting monitoring data in the continuous transmission mode is higher than that in the intermittent transmission mode. In addition, the third transmission mode includes a temporary continuous transmission mode, wherein the temporary continuous transmission mode is a "temporary" continuous transmission mode, which can be specifically reflected as follows: compared with the continuous transmission mode, the temporary continuous transmission mode also transmits data in a preset number of continuous transmission cycles, but said transmission cycles are less than the transmission cycles in the continuous transmission mode; or, compared with the intermittent transmission mode, the temporary continuous transmission mode also transmits data in some cycles in the preset number of continuous transmission cycles, and said some cycles may be continuous, and a number of said some cycles is greater than a number of cycle(s) for transmitting data in the intermittent transmission mode. The continuous transmission mode, the intermittent transmission mode, and the temporary continuous transmission mode can be respectively adapted to the scenarios corresponding to the first transmission mode, the second transmission mode, and the third transmission mode described above, and will not be described in detail here.

In an embodiment of this disclosure, the monitoring data transmitted in the continuous transmission mode includes real-time monitoring data; and the monitoring data transmitted in the temporary continuous transmission mode includes an analysis result of real-time monitoring data. In this embodiment, respective monitoring data transmitted in the continuous transmission mode and the temporary continuous transmission mode may be different. In the continuous transmission mode, the mobile monitoring device 100 transmits the real-time monitoring data to the monitoring device 200, and the monitoring device 200 can display such real-time data and execute a more complex algorithm to analyze the real-time data to obtain some analysis result data. In the temporary continuous transmission mode, after the mobile monitoring device 100 obtains real-time monitoring data, since the mobile monitoring device 100 does not need to transmit the real-time monitoring data in real time, the mobile monitoring device 100 can analyze the real-time monitoring data and transmit analysis result data to the monitoring device 200. The monitoring device 200 can directly display such analysis result data without having to perform complex algorithms itself or just need to perform some algorithms that are not performed on the mobile monitoring device 100.

The above exemplary shows the monitoring system 20 according to the embodiment of this disclosure. Based on the above description, after acquiring the monitoring data of the human body, the wearable mobile monitoring device in the monitoring system 20 according to the embodiment of this disclosure can transmit the monitoring data to the monitoring device which is separate from the human body through different transmission modes. These different transmission modes can satisfy a scenario with a high monitoring requirement, a scenario with a low power consumption requirement, and a scenario that requires temporary mode switching, so as to satisfy various complex scenarios while minimizing power consumption.

The following describes a data transmission method 600 which is provided according to another aspect of this disclosure in conjunction with FIG. 6. The method 600 is applied to the wearable mobile monitoring device described above. The data transmission process of the wearable mobile monitoring device has been described in detail above. Those skilled in the art can understand the specific details of the data transmission method 600 in combination with the above. For the sake of brevity, only some main steps of the data transmission method 600 are described here.

As shown in FIG. 6, the data transmission method 600 may include following steps.

In step S610, acquire a signal which represents at least one physiological sign parameter of a human body.

In step S620, obtain monitoring data based on the signal.

In step S630, transmit the monitoring data to a monitoring device which is separate from a human body, through a communicative connection which is established between the mobile monitoring device and the monitoring device.

Wherein, step S630 further includes step S631, step S632 and step S633.

In step S631, under a first preset condition, transmit, in a first transmission mode, the monitoring data which is acquired at a first acquisition frequency.

In step S632, under a second preset condition, transmit, in a second transmission mode, the monitoring data which is acquired at a second acquisition frequency.

In step S633, when the monitoring data is transmitted in the second transmission mode; if a third preset condition is detected, switch to a third transmission mode in which mode the monitoring data, which is acquired at a third acquisition frequency, is transmitted; and further switch back to the second transmission mode, when a transmission duration after switching to the third transmission mode reaches a first preset time threshold and the first preset condition is not detected.

Wherein a frequency for transmitting the monitoring data in the third transmission mode is higher than a frequency for transmitting the monitoring data in the second transmission mode, and/or the third acquisition frequency is higher than the second acquisition frequency.

In an embodiment of this disclosure, the third preset condition includes a user instruction for switching to the third transmission mode.

In an embodiment of this disclosure, the first preset condition includes: an indicator, which reflects a communication state between the mobile monitoring device and the monitoring device, satisfies a fourth preset condition, and/or an indicator, which reflects a health state of the human body, satisfies a fifth preset condition; the second preset condition includes: under the premise that no user instruction for switching a transmission mode is detected, an indicator, which reflects a communication state between the mobile monitoring device and the monitoring device, satisfies a sixth preset condition, and/or, under the premise that no user instruction for switching a transmission mode is detected, an indicator, which reflects a health state of the human body, satisfies a seventh preset condition.

In an embodiment of this disclosure, an indicator, which reflects a communication state between the mobile monitoring device and the monitoring device, satisfying a fourth preset condition, includes at least one of: a communicative connection between the mobile monitoring device and the monitoring device is a point-to-point wireless communicative connection; when the monitoring device is a monitor located inside a ward, a communication position of the mobile monitoring device is also located inside said ward; a communication distance between the mobile monitoring device and the monitoring device is less than a first preset distance threshold.

In an embodiment of this disclosure, an indicator, which reflects a communication state between the mobile monitoring device and the monitoring device, satisfying a sixth preset condition, includes at least one of: a communicative connection between the mobile monitoring device and the monitoring device is not point-to-point wireless communicative connection; when the monitoring device is a monitor located inside a ward, a communication position of the mobile monitoring device is located outside said ward; a communication distance between the mobile monitoring device and the monitoring device is not less than a second preset distance threshold.

In an embodiment of this disclosure, an indicator, which reflects a health state of the human body, satisfying a fifth preset condition, includes at least one of: a monitoring time for the human body by the mobile monitoring device does not reach a second preset time threshold, wherein the second preset time threshold is greater than the first preset time threshold; an early warning score of the human body based on the monitoring data is lower than a first preset score threshold; at least one item of the monitoring data has an abnormality at a current moment; at least one item of the monitoring data has no abnormality at a current moment, but has an abnormality within a preset time before said current moment, and a duration of the preset time is a third preset time threshold; an accident event for movement occurs to the human body.

In an embodiment of this disclosure, an indicator, which reflects a health state of the human body, satisfying a seventh preset condition, includes at least one of: a monitoring time for the human body by the mobile monitoring device reaches a fourth preset time threshold, wherein the fourth preset time threshold is greater than the first preset time threshold; an early warning score of the human body based on the monitoring data is higher than a second preset score threshold; the monitoring data has no abnormality; no accident event for movement occurs to the human body.

In an embodiment of this disclosure, the method also includes: outputting countdown indication information according to the first preset time threshold, so as to indicate to a user that the third transmission mode is about to be switched back to the second transmission mode; wherein a start moment of countdown in the countdown indication information is when the second transmission mode is switched to the third transmission mode.

In an embodiment of this disclosure, the method further includes: outputting countdown indication information according to the third preset time threshold, so as to indicate to a user that the first transmission mode is about to be switched to the second transmission mode; wherein a start moment of countdown in the countdown indication information is when the abnormality disappears.

In an embodiment of this disclosure, the countdown indication information is displayed on a display of the mobile monitoring device and/or a display of the monitoring device; and/or, the countdown indication information is played through a speaker of the mobile monitoring device and/or a speaker of the monitoring device.

In an embodiment of this disclosure, the monitoring device includes a monitor located inside a ward, and/or a central monitoring system.

In an embodiment of this disclosure, the first transmission mode includes a continuous transmission mode, the second transmission mode includes an intermittent transmission mode, and the third transmission mode includes a temporary continuous transmission mode, wherein: the continuous transmission mode refers to transmitting data in a preset number of continuous transmission cycles; the intermittent transmission mode refers to transmitting data only in a first part of transmission cycle(s) among a preset number of continuous transmission cycles; the temporary continuous transmission mode refers to transmitting data only in a second part of transmission cycles among a preset number of continuous transmission cycles, wherein a number of the second part of transmission cycles is greater than a number of the first part of transmission cycle(s).

In an embodiment of this disclosure, the monitoring data transmitted in the continuous transmission mode includes real-time monitoring data; the monitoring data transmitted in the temporary continuous transmission mode includes an analysis result of real-time monitoring data.

Based on the above description, after obtaining the monitoring data of the human body, the wearable mobile monitoring device in the data transmission method according to an embodiment of this disclosure is capable of transmitting the monitoring data to the monitoring device which is separate from the human body through different transmission modes. These different transmission modes are capable of satisfying a scenario with a high monitoring requirement, a scenario with a low power consumption requirement, and a scenario that requires temporary mode switching, so as to satisfy various complex scenarios while minimizing power consumption.

In addition, according to an embodiment of this disclosure, a storage medium is also provided, on which program instructions are stored. When executed by a computer or a processor, the program instructions are configured to execute the corresponding steps of the data transmission method according to an embodiment of this disclosure. The storage medium may include, for example, a memory card of a smart phone, a storage component of a tablet computer, a hard disk of a personal computer, a read-only memory (ROM), an erasable programmable read-only memory (EPROM), a portable compact disk read-only memory (CD-ROM), a USB memory, or any combination of the above storage media. The computer-readable storage medium may be any combination of one or more computer-readable storage mediums.

Although the exemplary embodiments are described here with reference to the accompanying drawings, it should be understood that the aforementioned exemplary embodiments are only illustrative and are not intended to limit the scope of this disclosure. Those skilled in the art can make various changes and modifications without deviating from the scope and spirit of this disclosure. All these changes and modifications are intended to be included within the scope of this disclosure as claimed in the attached claims.

Those skilled in the art can realize that the units and algorithm steps described in combination with this disclosure can be implemented in electronic hardware, or a combination of computer software and electronic hardware. Whether these functions are executed in hardware or software depends on the specific application and design constraints of the technical solution. Professional technicians may use different methods to implement the described functions for each specific application, but such implementation should not be considered beyond the scope of this disclosure.

In the several embodiments provided by this disclosure, it should be understood that the disclosed devices and methods can be implemented in other ways. For example, the device embodiments described above are only schematic. For example, the division of the units is only a logical functional division, and there may be other division methods in actual implementations. For example, multiple units or components can be combined or integrated into another device, or some characteristics can be ignored or not executed.

The disclosure provided here provides a large number of specific details. However, it can be understood that the embodiments of this disclosure can be practiced without these specific details. In some examples, well-known methods, structures, and techniques are not shown in detail to avoid blurring the understanding of this disclosure.

Similarly, it should be understood that in order to streamline this disclosure and assist in understanding one or more of the various aspects of this disclosure, in the description of exemplary embodiments of this disclosure, the various characteristics of this disclosure are sometimes grouped together into a single embodiment, diagram, or description thereof. However, the method of this disclosure should not be interpreted as reflecting the intention that the claimed protection of this disclosure requires more characteristics than those explicitly recorded in each claim. More precisely, as reflected in the corresponding claims, the inventive point is that the corresponding technical problem can be solved with less than all characteristics of a single disclosed embodiment. Therefore, the claims following the specific implementation method are explicitly incorporated into the specific implementation method, where each claim itself serves as a separate embodiment of this disclosure.

Those skilled in the art can understand that, except mutual exclusion between characteristics, any combination can be configured to combine all characteristics disclosed in this disclosure (including accompanying claims, abstract, and drawings), as well as all processes or units of any method or device so disclosed. Unless otherwise explicitly stated, each characteristic disclosed in this disclosure (including accompanying claims, abstract, and accompanying drawings) may be replaced by alternative characteristics that provide the same, equivalent, or similar purpose.

In addition, those skilled in the art can understand that although some embodiments described herein include certain characteristics rather than other characteristics included in other embodiments, the combination of characteristics of different embodiments means that they are within the scope of this disclosure and form different embodiments. For example, in the claims, any one of the claimed embodiments can be used in any combination.

The various component embodiments of this disclosure may be implemented in hardware, or in a software module running on one or more processors, or in a combination thereof. Those skilled in the art should understand that a microprocessor or a digital signal processor (DSP) may be used in practice to implement some or all of the functions of some modules according to the embodiments of this disclosure. This disclosure may also be implemented as a device program (e.g., a computer program and a computer program product) for executing a part or all of the methods described herein. Such a program for implementing this disclosure may be stored on a computer-readable storage medium, or may have a form of one or more signals. Such a signal may be downloaded from an internet website, or provided on a carrier signal, or in any other form.

It should be noted that the above-mentioned embodiments illustrate rather than limit this disclosure and that those skilled in the art will be able to design alternative embodiments without departing from the scope of the appended claims. In the claims, any reference signs placed between parentheses shall not be construed as limiting the claim. This disclosure can be implemented by means of hardware including several distinct elements, and by means of a suitably programmed computer. In a unit claim enumerating several apparatuses, several of these apparatuses may be embodied by one same hardware item. The usage of the words first, second, and third etc., do not indicate any order. These words can be interpreted as names.

The above description is only a specific implementation manner or an explanation of a specific embodiment of this disclosure, and the protection scope of this disclosure is not limited thereto. Any technician familiar with the technical field can easily think of changes or substitutions within the technical scope disclosed in this disclosure, which should be covered by the protection scope of this disclosure. The protection scope of this disclosure shall be based on the protection scope of the claims.

## Claims

1. A wearable mobile monitoring device, **characterized in that**, comprising a sensor, a processor and a wireless communication unit; wherein:
the sensor is configured to acquire a signal which represents at least one vital sign parameter of a human body;
the processor is configured to perform following operations:
obtaining monitoring data which corresponds to the signal, based on the signal which is acquired by the sensor; controlling the wireless communication unit to transmit the monitoring data to a monitoring device which is separate from the human body, through a communicative connection which is established between the wireless communication unit and the monitoring device;
wherein transmitting the monitoring data to the monitoring device comprises:
under a first preset condition, transmitting, in a first transmission mode, the monitoring data which corresponds to the signal which is acquired at a first acquisition frequency;
under a second preset condition, transmitting, in a second transmission mode, the monitoring data which corresponds to the signal which is acquired at a second acquisition frequency; wherein a frequency for transmitting the monitoring data in the first transmission mode is higher than a frequency for transmitting the monitoring data in the second transmission mode, and/or the first acquisition frequency is higher than the second acquisition frequency;
when the monitoring data is transmitted in the second transmission mode; if a third preset condition is detected, switching to a third transmission mode in which mode the monitoring data, which corresponds to the signal which is acquired at a third acquisition frequency, is transmitted, and further switching back to the second transmission mode, when a transmission duration after switching to the third transmission mode reaches a first preset time threshold and the first preset condition is not detected;
wherein a frequency for transmitting the monitoring data in the third transmission mode is higher than the frequency for transmitting the monitoring data in the second transmission mode, and/or the third acquisition frequency is higher than the second acquisition frequency.

2. The mobile monitoring device according to claim **1, characterized in that**, the third preset condition comprises a user instruction for switching to the third transmission mode.

3. The mobile monitoring device according to claim **1, characterized in that**,
the first preset condition comprises: an indicator, which reflects a communication state between the mobile monitoring device and the monitoring device, satisfies a fourth preset condition, and/or an indicator, which reflects a health state of the human body, satisfies a fifth preset condition; and/or
the second preset condition comprises: under the premise that no user instruction for switching a transmission mode is detected, an indicator, which reflects a communication state between the mobile monitoring device and the monitoring device, satisfies a sixth preset condition, and/or, under the premise that no user instruction for switching a transmission mode is detected, an indicator, which reflects a health state of the human body, satisfies a seventh preset condition.

4. The mobile monitoring device according to claim 3, **characterized in that**, an indicator, which reflects a communication state between the mobile monitoring device and the monitoring device, satisfying a fourth preset condition, comprises at least one of:
a communicative connection between the mobile monitoring device and the monitoring device is a point-to-point wireless communicative connection;
when the monitoring device is a monitor located inside a ward, a communication position of the mobile monitoring device is also located inside said ward; and
a communication distance between the mobile monitoring device and the monitoring device is less than a first preset distance threshold.

5. The mobile monitoring device according to claim 3, **characterized in that**, an indicator, which reflects a communication state between the mobile monitoring device and the monitoring device, satisfying a sixth preset condition, comprises at least one of:
a communicative connection between the mobile monitoring device and the monitoring device is not a point-to-point wireless communicative connection;
when the monitoring device is a monitor located inside a ward, a communication position of the mobile monitoring device is located outside said ward; and
a communication distance between the mobile monitoring device and the monitoring device is not less than a second preset distance threshold.

6. The mobile monitoring device according to claim 3, **characterized in that**, an indicator, which reflects a health state of the human body, satisfying a fifth preset condition, comprises at least one of:
a monitoring time for the human body by the mobile monitoring device does not reach a second preset time threshold, wherein the second preset time threshold is greater than the first preset time threshold;
an early warning score of the human body based on the monitoring data is lower than a first preset score threshold;
at least one item of the monitoring data has an abnormality at a current moment;
at least one item of the monitoring data has no abnormality at a current moment, but has an abnormality within a preset time before said current moment, and a duration of the preset time is a third preset time threshold; and
an accident event for movement occurs to the human body.

7. The mobile monitoring device according to claim 3, **characterized in that**, an indicator, which reflects a health state of the human body, satisfying a seventh preset condition, comprises at least one of:
a monitoring time for the human body by the mobile monitoring device reaches a fourth preset time threshold, wherein the fourth preset time threshold is greater than the first preset time threshold;
an early warning score of the human body based on the monitoring data is higher than a second preset score threshold;
the monitoring data has no abnormality; and
no accident event for movement occurs to the human body.

8. The mobile monitoring device according to claim 1, **characterized in that**, the processor is further configured to perform a following operation:
outputting countdown indication information according to the first preset time threshold, so as to indicate to a user that the third transmission mode is about to be switched back to the second transmission mode; wherein a start moment of countdown in said countdown indication information is when the second transmission mode is switched to the third transmission mode.

9. The mobile monitoring device according to claim 6, **characterized in that**, the processor is further configured to perform a following operation:
outputting countdown indication information according to the third preset time threshold, so as to indicate to a user that the first transmission mode is about to be switched to the second transmission mode; wherein a start moment of countdown in said countdown indication information is when the abnormality disappears.

10. The mobile monitoring device according to claim 8 or 9, **characterized in that**, the countdown indication information is displayed on a display of the mobile monitoring device and/or a display of the monitoring device; and/or
the countdown indication information is played through a speaker of the mobile monitoring device and/or a speaker of the monitoring device.

11. The mobile monitoring device according to claim 1, **characterized in that**, the monitoring device comprises a monitor located inside a ward, and/or a central monitoring system.

12. The mobile monitoring device according to claim 1, **characterized in that**, the first transmission mode comprises a continuous transmission mode, the second transmission mode comprises an intermittent transmission mode, and the third transmission mode comprises a temporary continuous transmission mode, wherein:
the continuous transmission mode refers to transmitting data in a preset number of continuous transmission cycles;
the intermittent transmission mode refers to transmitting data only in a first part of transmission cycle(s) among a preset number of continuous transmission cycles;
the temporary continuous transmission mode refers to transmitting data only in a second part of transmission cycles among a preset number of continuous transmission cycles, wherein a number of the second part of transmission cycles is greater than a number of the first part of transmission cycle(s).

13. The mobile monitoring device according to claim 11, **characterized in that**,
the monitoring data transmitted in the continuous transmission mode comprises real-time monitoring data; and/or
the monitoring data transmitted in the temporary continuous transmission mode comprises analysis result(s) of real-time monitoring data.

14. A monitoring system, **characterized in that**, comprising a wearable mobile monitoring device which is worn on a human body, and a monitoring device which is separate from the human body;
wherein the mobile monitoring device is configured to detect monitoring data of the human body;
the monitoring device is configured to acquire and display the monitoring data;
the mobile monitoring device is further configured to perform following operations:
transmitting the monitoring data to the monitoring device through a communicative connection which is established between the mobile monitoring device and the monitoring device; wherein transmitting the monitoring data to the monitoring device, comprises:
under a first preset condition, transmitting, in a first transmission mode, the monitoring data which is acquired at a first acquisition frequency;
under a second preset condition, transmitting, in a second transmission mode, the monitoring data which is acquired at a second acquisition frequency; wherein a frequency for transmitting the monitoring data in the first transmission mode is higher than a frequency for transmitting the monitoring data in the second transmission mode, and/or the first acquisition frequency is higher than the second acquisition frequency;
when the monitoring data is transmitted in the second transmission mode; if a third preset condition is detected, switching to a third transmission mode in which mode the monitoring data, which is acquired at a third acquisition frequency, is transmitted, and further switching back to the second transmission mode, when a transmission duration after switching to the third transmission mode reaches a first preset time threshold and the first preset condition is not detected;
wherein a frequency for transmitting the monitoring data in the third transmission mode is higher than the frequency for transmitting the monitoring data in the second transmission mode, and/or the third acquisition frequency is higher than the second acquisition frequency.

15. The monitoring system according to claim 14, **characterized in that**, the third preset condition comprises a user instruction for switching to the third transmission mode.

16. The monitoring system according to claim 14, **characterized in that**,
the first preset condition comprises: an indicator, which reflects a communication state between the mobile monitoring device and the monitoring device, satisfies a fourth preset condition, and/or an indicator, which reflects a health state of the human body, satisfies a fifth preset condition;
the second preset condition comprises: under the premise that no user instruction for switching a transmission mode is detected, an indicator, which reflects a communication state between the mobile monitoring device and the monitoring device, satisfies a sixth preset condition, and/or, under the premise that no user instruction for switching a transmission mode is detected, an indicator, which reflects a health state of the human body, satisfies a seventh preset condition.

17. The monitoring system according to claim 16, **characterized in that**, an indicator, which reflects a communication state between the mobile monitoring device and the monitoring device, satisfying a fourth preset condition, comprises at least one of:
a communicative connection between the mobile monitoring device and the monitoring device is a point-to-point wireless communicative connection;
when the monitoring device is a monitor located inside a ward, a communication position of the mobile monitoring device is also located inside said ward; and
a communication distance between the mobile monitoring device and the monitoring device is less than a first preset distance threshold.

18. The monitoring system according to claim 16, **characterized in that**, an indicator, which reflects a communication state between the mobile monitoring device and the monitoring device, satisfying a sixth preset condition, comprises at least one of:
a communicative connection between the mobile monitoring device and the monitoring device is not a point-to-point wireless communicative connection;
when the monitoring device is a monitor located inside a ward, a communication position of the mobile monitoring device is located outside said ward; and
a communication distance between the mobile monitoring device and the monitoring device is not less than a second preset distance threshold.

19. The monitoring system according to claim 16, **characterized in that**, an indicator, which reflects a health state of the human body, satisfying a fifth preset condition, comprises at least one of:
a monitoring time for the human body by the mobile monitoring device does not reach a second preset time threshold, wherein the second preset time threshold is greater than the first preset time threshold;
an early warning score of the human body based on the monitoring data is lower than a first preset score threshold;
at least one item of the monitoring data has an abnormality at a current moment;
at least one item of the monitoring data has no abnormality at a current moment, but has an abnormality within a preset time before said current moment, and a duration of the preset time is a third preset time threshold; and
an accident event for movement occurs to the human body.

20. The monitoring system according to claim 16, **characterized in that**, an indicator, which reflects a health state of the human body, satisfying a seventh preset condition, comprises at least one of:
a monitoring time for the human body by the mobile monitoring device reaches a fourth preset time threshold, wherein the fourth preset time threshold is greater than the first preset time threshold;
an early warning score of the human body based on the monitoring data is higher than a second preset score threshold;
the monitoring data has no abnormality; and
no accident event for movement occurs to the human body.

21. The monitoring system according to claim 14, **characterized in that**, the mobile monitoring device is further configured to perform a following operation:
outputting countdown indication information according to the first preset time threshold, so as to indicate to a user that the third transmission mode is about to be switched back to the second transmission mode; wherein a start moment of countdown in said countdown indication information is when the second transmission mode is switched to the third transmission mode.

22. The monitoring system according to claim 19, **characterized in that**, the mobile monitoring device is further configured to perform a following operation:
outputting countdown indication information according to the third preset time threshold, so as to indicate to a user that the first transmission mode is about to be switched to the second transmission mode; wherein a start moment of countdown in said countdown indication information is when the abnormality disappears.

23. The monitoring system according to claim 21 or 22, **characterized in that**, the countdown indication information is displayed on a display of the mobile monitoring device and/or a display of the monitoring device; and/or
the countdown indication information is played through a speaker of the mobile monitoring device and/or a speaker of the monitoring device.

24. The monitoring system according to claim 14, **characterized in that**, the monitoring device comprises a monitor located inside a ward, and/or a central monitoring system.

25. The monitoring system according to claim 14, **characterized in that**, the first transmission mode comprises a continuous transmission mode, the second transmission mode comprises an intermittent transmission mode, and the third transmission mode comprises a temporary continuous transmission mode, wherein:
the continuous transmission mode refers to transmitting data in a preset number of continuous transmission cycles;
the intermittent transmission mode refers to transmitting data only in a first part of transmission cycle(s) among a preset number of continuous transmission cycles;
the temporary continuous transmission mode refers to transmitting data only in a second part of transmission cycles among a preset number of continuous transmission cycles, wherein a number of the second part of transmission cycles is greater than a number of the first part of transmission cycle(s).

26. The monitoring system according to claim 25, **characterized in that**,
the monitoring data transmitted in the continuous transmission mode comprises real-time monitoring data;
the monitoring data transmitted in the temporary continuous transmission mode comprises analysis result(s) of real-time monitoring data.

27. A data transmission method which is applied to a wearable mobile monitoring device, **characterized in that**, the method comprises:
acquiring a signal which represents at least one physiological sign parameter of a human body;
obtaining monitoring data based on the signal; and
transmitting the monitoring data to a monitoring device which is separate from the human body, through a communicative connection which is established between the mobile monitoring device and the monitoring device;
wherein transmitting the monitoring data to the monitoring device, comprises:
under a first preset condition, transmitting, in a first transmission mode, the monitoring data which is acquired at a first acquisition frequency;
under a second preset condition, transmitting, in a second transmission mode, the monitoring data which is acquired at a second acquisition frequency; wherein a frequency for transmitting the monitoring data in the first transmission mode is higher than a frequency for transmitting the monitoring data in the second transmission mode, and/or the first acquisition frequency is higher than the second acquisition frequency;
when the monitoring data is transmitted in the second transmission mode; if a third preset condition is detected, switching to a third transmission mode in which mode the monitoring data, which is acquired at a third acquisition frequency, is transmitted, and further switching back to the second transmission mode, when a transmission duration after switching to the third transmission mode reaches a first preset time threshold and the first preset condition is not detected;
wherein a frequency for transmitting the monitoring data in the third transmission mode is higher than the frequency for transmitting the monitoring data in the second transmission mode, and/or the third acquisition frequency is higher than the second acquisition frequency.

28. The method according to claim 27, **characterized in that**, the third preset condition comprises a user instruction for switching to the third transmission mode.

29. The method according to claim 27, **characterized in that**,
the first preset condition comprises: an indicator, which reflects a communication state between the mobile monitoring device and the monitoring device, satisfies a fourth preset condition, and/or an indicator, which reflects a health state of the human body, satisfies a fifth preset condition;
the second preset condition comprises: under the premise that no user instruction for switching a transmission mode is detected, an indicator, which reflects a communication state between the mobile monitoring device and the monitoring device, satisfies a sixth preset condition, and/or, under the premise that no user instruction for switching a transmission mode is detected, an indicator, which reflects a health state of the human body, satisfies a seventh preset condition.

30. The method according to claim 29, **characterized in that**, an indicator, which reflects a communication state between the mobile monitoring device and the monitoring device, satisfying a fourth preset condition, comprises at least one of:
a communicative connection between the mobile monitoring device and the monitoring device is a point-to-point wireless communicative connection;
when the monitoring device is a monitor located inside a ward, a communication position of the mobile monitoring device is also located inside said ward; and
a communication distance between the mobile monitoring device and the monitoring device is less than a first preset distance threshold.

31. The method according to claim 29, **characterized in that**, an indicator, which reflects a communication state between the mobile monitoring device and the monitoring device, satisfying a sixth preset condition, comprises at least one of:
a communicative connection between the mobile monitoring device and the monitoring device is not a point-to-point wireless communicative connection;
when the monitoring device is a monitor located inside a ward, a communication position of the mobile monitoring device is located outside said ward; and
a communication distance between the mobile monitoring device and the monitoring device is not less than a second preset distance threshold.

32. The method according to claim 29, **characterized in that**, an indicator, which reflects a health state of the human body, satisfying a fifth preset condition, comprises at least one of:
a monitoring time for the human body by the mobile monitoring device does not reach a second preset time threshold, wherein the second preset time threshold is greater than the first preset time threshold;
an early warning score of the human body based on the monitoring data is lower than a first preset score threshold;
at least one item of the monitoring data has an abnormality at a current moment;
at least one item of the monitoring data has no abnormality at a current moment, but has an abnormality within a preset time before said current moment, and a duration of the preset time is a third preset time threshold; and
an accident event for movement occurs to the human body.

33. The method according to claim 29, **characterized in that**, an indicator, which reflects a health state of the human body, satisfying a seventh preset condition, comprises at least one of:
a monitoring time for the human body by the mobile monitoring device reaches a fourth preset time threshold, wherein the fourth preset time threshold is greater than the first preset time threshold;
an early warning score of the human body based on the monitoring data is higher than a second preset score threshold;
the monitoring data has no abnormality; and
no accident event for movement occurs to the human body.

34. The method according to claim 27, **characterized in that**, further comprising:
outputting countdown indication information according to the first preset time threshold, so as to indicate to a user that the third transmission mode is about to be switched back to the second transmission mode; wherein a start moment of countdown in said countdown indication information is when the second transmission mode is switched to the third transmission mode.

35. The method according to claim 32, **characterized in that**, further comprising:
outputting countdown indication information according to the third preset time threshold, so as to indicate to a user that the first transmission mode is about to be switched to the second transmission mode; wherein a start moment of countdown in said countdown indication information is when the abnormality disappears.

36. The method according to claim 34 or 35, **characterized in that**, the countdown indication information is displayed on a display of the mobile monitoring device and/or a display of the monitoring device; and/or
the countdown indication information is played through a speaker of the mobile monitoring device and/or a speaker of the monitoring device.

37. The method according to claim 27, **characterized in that**, the monitoring device comprises a monitor located inside a ward, and/or a central monitoring system.

38. The method according to claim 27, **characterized in that**, the first transmission mode comprises a continuous transmission mode, the second transmission mode comprises an intermittent transmission mode, and the third transmission mode comprises a temporary continuous transmission mode, wherein:
the continuous transmission mode refers to transmitting data in a preset number of continuous transmission cycles;
the intermittent transmission mode refers to transmitting data only in a first part of transmission cycle(s) among a preset number of continuous transmission cycles;
the temporary continuous transmission mode refers to transmitting data only in a second part of transmission cycles among a preset number of continuous transmission cycles, wherein a number of the second part of transmission cycles is greater than a number of the first part of transmission cycle(s).

39. The method according to claim 38, **characterized in that**,
the monitoring data transmitted in the continuous transmission mode comprises real-time monitoring data;
the monitoring data transmitted in the temporary continuous transmission mode comprises analysis result(s) of real-time monitoring data.
